(19) Europäisches Patentamt
European Patent Office
Office européen des brevets

(11) Veröffentlichungsnummer: **0 435 902 B1**

(12) # EUROPÄISCHE PATENTSCHRIFT

(45) Veröffentlichungstag der Patentschrift: **20.12.95**

(51) Int. Cl.[6]: **C07D 295/084**, A61K 31/495

(21) Anmeldenummer: **89910438.4**

(22) Anmeldetag: **12.09.89**

(86) Internationale Anmeldenummer: **PCT/EP89/01058**

(87) Internationale Veröffentlichungsnummer: **WO 90/03371 (05.04.90 90/08)**

Die Akte enthält technische Angaben, die nach dem Eingang der Anmeldung eingereicht wurden und die nicht in dieser Patentschrift enthalten sind.

(54) **2-HYDROXY-3-PHENOXY-PROPYL-SUBSTITUIERTE PIPERAZINE, IHRE HERSTELLUNG UND VERWENDUNG**

(30) Priorität: **21.09.88 DE 3831993**

(43) Veröffentlichungstag der Anmeldung: **10.07.91 Patentblatt 91/28**

(45) Bekanntmachung des Hinweises auf die Patenterteilung: **20.12.95 Patentblatt 95/51**

(84) Benannte Vertragsstaaten: **AT BE CH DE FR GB IT LI LU NL SE**

(56) Entgegenhaltungen:
**EP-A- 0 025 111**
**EP-A- 0 068 324**
**EP-A- 0 243 886**
**FR-A- 2 112 509**
**US-A- 3 133 925**

(73) Patentinhaber: **BASF Aktiengesellschaft**
**Carl-Bosch-Strasse 38**
**D-67063 Ludwigshafen (DE)**

(72) Erfinder: **TREIBER, Hans-Jörg**
**Sperberweg 1**
**D-6835 Brühl (DE)**
Erfinder: **HOFMANN, Hans, Peter**
**Untere Hart 12**
**D-6703 Limburgerhof (DE)**
Erfinder: **SZABO, Laszlo**
**Kastellweg 10**
**D-6900 Heidelberg (DE)**
Erfinder: **UNGER, Liliane**
**Waltraudenstrasse 14**
**D-6700 Ludwigshafen (DE)**
Erfinder: **RASCHACK, Manfred**
**Donnersbergstrasse 7**
**D-6714 Weisenheim am Sand (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 2-Hydroxy-3-phenoxy-propyl-substituierte Piperazine, Verfahren zu deren Herstellung und deren Verwendung als Arzneimittel.

Es sind bereits zahlreiche verschiedenartige Piperazine bekannt. So sind z. B. aus der Reihe der Benzhydryl- und Diarylbutylpiperazine Flunarizin (DE-A-1 570 001) und Lidoflazin (DE-B-1 929 330) als Therapeutika von Herz-Kreislauf- und cerebrovaskulären Erkrankungen bekannt. Ihre Wirkungsweise beruht auf einer Hemmung des Calciumeinstroms in die Zelle.

Dieser Wirkmechanismus gilt auch für die in der DE-A-3.600.390 beschriebenen ω-Phenoxy-alkyl-piperazine. Das therapeutische Ziel dieser Verbindungen ist, ebenso wie das der in der DE-A-3.326.148 beschriebenen ßenzhydryl-phenylalkanol-substituierten Piperazine, die Behandlung cerebraler Kreislauferkrankungen.

N-Arylpiperazinalkanamide mit Diarylbutylsubstituenten am Piperazinring (vgl. EP-A-68544) verbessern die Durchblutung des Herzens und schützen es vor den Folgen einer Ischaemie, Anoxie oder Hypoxie.

Weiter bekannt sind 3-Aryloxy-2-Propanol-derivate des Phenylpiperazins, die jedoch keine calciumantagonistischen oder antihypoxischen Wirkungen aufweisen. (Arzneim. Forsch. (1978) 28 241-246).

Es wurde nun gefunden, daß 2-Hydroxy-3-phenoxy-propyl-substituierte Piperazine der Formel I

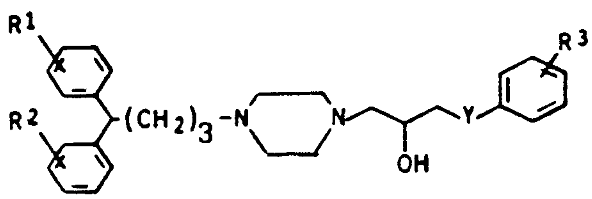

I

worin

$R^1$ und $R^2$ Wasserstoff-, Fluor- oder Chloratome,

$R^3$ ein Fluor- oder Chloratom und

Y ein Sauerstoff- oder Schwefelatom,

bedeuten, starke Calcium-Antagonisten mit hoher cerebral-antihypoxischer Wirkung darstellen, welche die regionale Hirndurchblutung signifikant verbessern.

Die erfindungsgemäßen Verbindungen erscheinen daher für die Therapie von Herz-Kreislauf-Erkrankungen im allgemeinen und insbesondere zur Behandlung von akuten und chronischen Sauerstoffmangel-Zustände des Gehirns geeignet. Hierunter sind akute hypoxische bzw. ischämische Zustände zu verstehen, die z.B. infolge Hirninfarkt, Schädel-Hirn-Trauma oder Gefäßspasmen sowie infolge von Herz-Kreislauf-Versagen, z.B. bei Herzstillstand, kardialen Arrhythmien oder Kreislaufschock auftreten. Als Krankheitsbilder mit chronischen Sauerstoffmangel-Zuständen kommen z.B. in Betracht: transitorische ischämische Attacken (TIAs) und prolongierte reversible ischämische neurologische Defizite (PRINDs), aber auch Multiinfarktdemenz und atherosklerotische Demenz, außerdem Migräne und Epilepsien.

Als physikalisch verträgliche Säuren kommen in Betracht: Anorganische Säuren wie Salzsäure, Schwefelsäure, Phosphorsäure, Salpetersäure oder organische Säuren wie Weinsäure, Milchsäure, Apfelsäure, Zitronensäure, Maleinsäure, Fumarsäure, Oxalsäure, Essigsäure, Glukonsäure oder Schleimsäure.

In der Formel I ist Y insbesondere ein Sauerstoffatom. Die Substituenten $R^1$, $R^2$ und $R^3$ stehen insbesondere in der 3- oder 4-Stellung der Phenylringe. Die 4-Stellungen sind bevorzugt.

Die Verbindungen der Formel I besitzen in dem Strukturelement des Aryloxypropanols ein Chiralitätszentrum. Sie liegen daher in Form optischer Antipoden (Enantiomere) vor. Die Racemate können nach gebräuchlichen Methoden durch Salzbildung mit chiralen Hilfskomponenten wie Dibenzoylweinsäure, fraktionierte Kristallisation und anschließende Freisetzung der Basen aus den Salzen oder auch synthetisch aus geeigneten chiralen Vorstufen erhalten werden.

Die Erfindung betrifft weiterhin Verfahren zur Herstellung der Verbindungen der Formel I, die dadurch gekennzeichnet sind, daß man entweder

a) eine Verbindung der Formel II

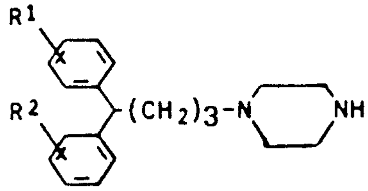

II

in welcher $R^1$ und $R^2$ die angegebene Bedeutung haben, mit einem Epoxid der Formel III,

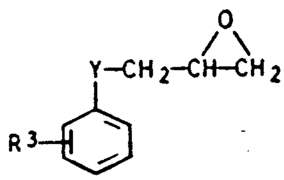

III

worin Y und $R^3$ die angegebene Bedeutung haben, oder
b) eine Verbindung der Formel IV

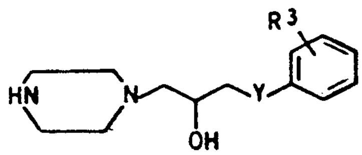

IV

worin Y und $R^3$ die angegebene Bedeutung haben,
mit einer Verbindung der Formel V

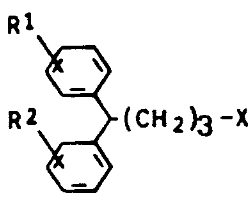

V

wobei $R^1$ und $R^2$ die angegebene Bedeutung haben und X einen reaktionsfähigen Säurerest bedeutet, umsetzt. Die so erhaltenen Verbindungen können anschließend mit physiologisch verträglichen Säuren in ihre Salze überführt werden.

Die an sich bekannte Umsetzung von Epoxiden der Formel III mit sekundären Aminen der Formel II wird zweckmäßigerweise in einem Lösungsmittel wie Acetonitril, Dimethylformamid, Tetrahydrofuran oder einem niederen Alkohol vorzugsweise Methanol oder Ethanol bei erhöhten Temperaturen (30 - 120 °C) vorzugsweise bei Siedetemperaturen des Lösungsmittels, durchgeführt.

Verfahren b) wird vorzugsweise in einem polaren organischen Lösungsmittel wie Alkohlen z. B. Methanol, Ethanol, Isopropanol oder einem niederen Keton, vorzugsweise Aceton, Methylethylketon oder Methylisobutylketon oder in Dimethylformamid. Dimethylsulfoxid, Acetonitril, gegebenenfalls auch in einem Kohlenwasserstoff wie Toluol vorteilhaft in Gegenwart einer Hilfsbase zum Abfangen der sich bildenden Säure wie beispielsweise Natriumcarbonat, Kaliumcarbonat, Calciumcarbonat, Triethylamin oder Pyridin bei erhöhter Temperatur, vorzugsweise zwischen 20 und 120 °C durchgeführt. Als reaktionsfähige Säurereste X eignen sich Chlor-, Brom- oder Iodatome sowie Sulfonsäuregruppen, vorzugsweise Methansulfonyl-, Benzolsulfonyl-, p-Toluolsulfonyl- oder Trifluormethansulfonylreste.

Die Ausgangsverbindungen der Formel II bis V sind literaturbekannt oder lassen sich in an sich bekannter Weise analog herstellen. So erhält man durch Umsetzung von optisch aktivem (R)-oder (S)-Epichlorhydrin mit Phenolen oder Thiophenolen der Formel VI

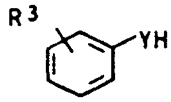

VI

worin Y und $R^3$ die angegebenen Bedeutungen haben, optisch aktive Epoxide der Formel III, durch deren Umsetzung mit den Verbindungen der Formel II optisch aktive Verbindungen der Formel I erhalten werden können.

Die erfindungsgemäßen Verbindungen können in üblicher Weise oral, rektal oder parenteral (subkutan, intravenös, intramuskulär, transdermal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen 0,1 und 20 mg/kg Körpergewicht bei oraler Gabe und zwischen 0,01 und 2 mg/kg Körpergewicht bei parenteraler Gabe. Im Normalfall werden mit täglichen Dosen von 10 - 100 mg oral und 1 - 10 mg parenteral zufriedenstellende Ergebnisse erzielt.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen, Salben, Cremes, Sprays oder transdermale therapeutische Systeme. Diese werden in üblicher Weise hergestellt. Die Wirkstoffe können dabei mit den üblichen galenischen Hilsmitteln wie Tablettenbindern, Füllstoffen, Konservierungsstoffen, Tablettensprengmitteln, Fließreguliermitteln, Weichmachern, Netzmitteln, Dispergiermitteln, Emulgatoren, Lösungsmitteln, Retardierungsmitteln Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 75 Gewichtsprozent.

Beispiele

1. 1-(4,4-Bis(4-fluorphenyl)butyl]-4-[2-hydroxy-3(4-chlorphenoxy)propyl]piperazin

6,6 g (0,02 mol) 1-[4,4-Bis(4-fluorphenyl)]butylpiperazin und 3,7 g (0,02 mol) 3-(4-Chlorphenoxy)-1,2-epoxipropan wurden in 100 ml Ethanol 2 h zum Sieden unter Rückfluß erhitzt. Nach dem Abkühlen wurde das Lösungsmittel unter vermindertem Druck abdestilliert. Der ölige Rückstand mit etherischer Salzsäure versetzt, der Niederschlag abgesaugt und das Produkt mit Isopropanol-Wasser umkristallisiert.
Ausbeute: 5,2 g
Di-Hydrochlorid $C_{29}H_{33}ClF_2N_2O_2$ • 2 HCl, Fp. 205-211 °C.

Analog wurden hergestellt:

2. 1-[4,4-Bis(4-Fluorphenyl)butyl]-4-[2-hydroxy-3(4-fluorphenoxy)propyl]piperazin, Di-Hydrochlorid $C_{29}H_{33}F_3N_2O_2$ • 2 HCl, Fp. 225-227 °C,

3. 1-[4,4-Bis(4-fluorphenyl)butyl]-4-[2-hydroxy-3-(c-Chlorphenoxy)propyl]piperazin, Di-Hydrochlorid $C_{29}H_{33}ClF_2N_2O_2$ • 2HCl, Fp. 205-211 °C

4. 1-[4,4-Diphenylbutyl]-4-[2-hydroxy-3-(4-fluorphenoxy)propyl]piperazin, Di-Hydrochlorid $C_{29}H_{35}FN_2O_2$ • 2HCl, Fp. 198-201 °C

5. 1-[4,4-Bis(3-fluorphenyl)butyl]-4-[2-hydroxy-3-(4-fluorphenoxy)propyl]piperazin, Di-Hydrochlorid $C_{24}H_{33}F_3N_2O_2$ • 2HCl, Fp. 222-225 °C

6. 1-[4,4-Bis(4-chlorphenyl)butyl]-4-[2-hydroxy-3-(4-chlorphenoxy)propyl]piperazin, Di-Hydrochlorid $C_{29}H_{33}Cl_3N_2O_2$ • 2HCl, Fp. 190-196 °C

Durch Umsetzung der Enantiomeren 3-(4-Fluorphenoxy)-1,2-epoxy-propane (erhalten durch Synthese aus 4-Fluorphenol und (R)- bzw. (S)-Epichlorhydrin) mit 1-[4,4-Bis(4-fluorphenyl)butyl]piperazin analog Beispiel 1 wurden erhalten:

7. (-)-1[4,4-Bis(4-fluorphenyl)butyl]-4-[2-hydroxy-3(4-fluorphenoxy)propyl]piperazin, Di-Hydrochlorid $C_{29}H_{33}F_3N_2O_2$ • 2 HCl, Fp. 208 - 216 °C,

$$[\alpha]^{20}_{589\ nm} = -9{,}4^{\circ} \quad \text{(Methanol, 10 mg/ml)},$$

8. (+)-1[4,4-Bis(4-fluorphenyl)butyl]-4-[2-hydroxy-3(4-fluorphenoxy)propyl]piperazin, Di-Hydrochlorid $C_{29}H_{33}F_3N_2O_2 \cdot 2\ HCl$, Fp. 208 -216 °C,

$$[\alpha]^{20}_{589\ nm} = -9{,}4^{\circ} \quad \text{(Methanol, 10 mg/ml)},$$

9. Analog Beispiel 1 erhielt man durch Umsetzung von 1-[4,4-Bis(4-fluorphenyl)butyl]piperazin mit 3-(4-Fluor-thiophenoxy)1,2-epoxipropan (erhalten durch Umsetzung von 4-Fluor-thiophenol mit Epichlorhydrin)-1-[4,4-Bis(4-Fluorphenyl)butyl]-4-[2-hydroxy-3-(4-fluor-thiophenoxy)propyl]piperazin, Di-Hydrochlorid $C_{29}H_{33}F_3N_2OS \cdot 2HCl$, Fp. 212-215 °C.

Die Wirkung der neuen Substanzen wurde wie folgt gemessen:

1. Schutz gegen akute zerebrale Hypoxie

Weibliche Mäuse (Gewicht 22-28 g) wurden in ein Glasrohr (Durchmesser 6 cm) gesetzt, durch welches ein Gemisch aus 3,5 Teilen Sauerstoff und 96,5 Teilen Stickstoff geleitet wurde. Nicht behandelte Kontrolltiere überleben in einem solchen Glasrohr im Mittel 138 sec. Ermittelt wurde die Dosis an Wirksubstanzen, nach deren intraperitonealer Gabe die Überlebenszeit der Tiere um 50 % steigt.

2. Schutz gegen globale zerebrale Ischämie

Weibliche Mäuse (Gewicht 24-27 g) erhielten die Wirksubstanz intraperitoneal vor Gabe von 0,1 ml einer 80 %igen Lösung (g/l) von $MgCl_2 \cdot 6H_2O$. Nicht vorbehandelte Kontrolltiere überleben nach Gabe von $MgCl_2$ im Mittel 24 sec. Ermittelt wurde die Dosis an Wirksubstanzen, die die Überlebenszeit der Tiere um 50 % gegenüber Placebo-behandelten Tieren erhöht.

3. Affinität der Prüfsubstanzen zum Calcium-Kanal.

Die Affinität der Prüfsubstanzen zum Calcium-Kanal wurde durch Hemmung der spezifischen (S)-$^3$H-Devapamil-Bindung an Meerschweinchen-Skelettmuskel-Membranen bestimmt (FEBS Lett. 176, 371 (1984)). Dazu wurden die Membranen mit einer festen Konzentration von 1 nM (S)-$^3$H-Devapamil und steigenden Konzentrationen an Prüfsubstanz in 50 mM Tris-HCl/0,1 mM Phenylmethylsulfonylfluorid, (pH 7,4) 60 Minuten bei 20 °C inkubiert. Die unspezifische Bindung wurde mit $10^{-6}$ M (S)-Devapamil bestimmt. Anschließend wurden die Ansätze über Glasfaserfilter filtriert und die Menge des auf dem Filter zurückgehaltenen (S)-$^3$H-Devapamils mittels Flüssigkeitszintillationsmessung bestimmt. Die Ki-Werte wurden durch nichtlineare Regressionsanalyse ermittelt.

Die folgende Tabelle zeigt die in den oben genannten Tests erhaltenen Ergebnissen. Als Vergleichssubstanzen dienten die Substanzen Flunarizin und Lidoflazin, die Wirkstoffe von Handelsprodukten sind.

Tabelle

| Substanz des Beispiels Nr. | Versuch 1 mg/kg i.p. ED 50 % | Versuch 2 mg/kg i.p. ED 50 % | Versuch 3 Ki (nM) |
|---|---|---|---|
| 1 | 11 | 27 | 8 |
| 2 | 11 | 20 | 9,1 |
| 3 | 6,9 | 39 | 10 |
| 4 | 6,0 | 8,8 | 15 |
| 5 | 14 | 29 | 22 |
| 7 | 1,6 | 13 | 9 |
| 8 | 4,0 | 17 | 10 |
| 9 | 9,0 | 28 | 9 |
| Flunarizin | > 100 | 26,5 | 288 |
| Lidoflazin | 10 | > 46 | 20 |

**Patentansprüche**

1. 2-Hydroxy-3-phenoxy-propyl-substituierte Piperazine der Formel I

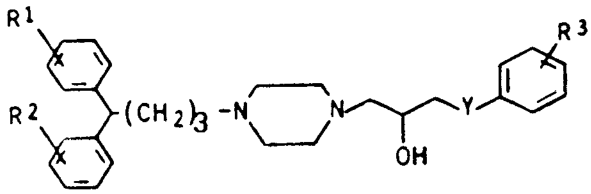

I

worin
   $R^1$ und $R^2$ Wasserstoff-, Fluor- oder Chloratome,
   $R^3$ ein Fluor- oder Chloratom und
   Y ein Sauerstoff- oder Schwefelatom
bedeuten,
sowie deren Salze mit physiologisch verträglichen Säuren.

2. 1-[4,4-Bis(4-fluorphenyl)butyl]-4-[2-hydroxy-3-(4-fluorphenoxy)propyl]piperazin

3. (-)-1-[4,4-Bis(4-fluorphenyl)butyl]-4-[2-hydroxy-3-[4-fluorphenoxy)propyl]piperazin

4. (+)-1-[4,4-Bis(4-fluorphenyl)butyl]-4-[2-hydroxy-3-(4-fluorphenoxy)propyl]piperazin

5. Verfahren zur Herstellung der Verbindungen der Formel I gemäß Anspruch 1, dadurch gekennzeichnet, daß man
   a) eine Verbindung der Formel II

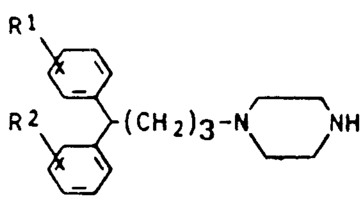

II

in welcher $R^1$ und $R^2$ die angegebene Bedeutung haben, mit einem Epoxid der Formel III,

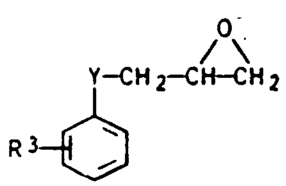

III

worin Y und $R^3$ die angegebene Bedeutung haben, oder

b) ein Piperazin der Formel IV

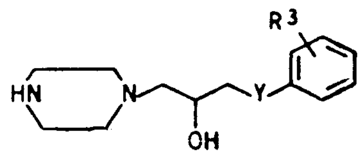

IV

worin Y und $R^3$ die angegebene Bedeutung haben, mit einer Verbindung der Formel V

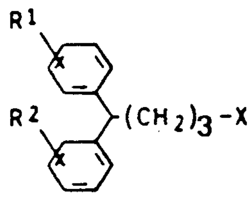

V

wobei $R^1$ und $R^2$ die angegebenen Bedeutung haben und X einen reaktionsfähigen Säurerest bedeutet, umsetzt und die so erhaltenen Verbindungen gegebenenfalls in ihre Salze mit physiologisch verträglichen Säuren überführt.

6. Arzneimittel enthaltend eine Verbindung der Formel I.

7. Verwendung einer Verbindung der Formel I zur Herstellung eines Arzneimittels zur Behandlung von Herz-Kreislauf-Erkrankungen und Sauerstoffmangelzuständen vitaler Organe.

## Claims

1. A 2-hydroxy-3-phenoxy-propyl-substituted piperazine of the formula I

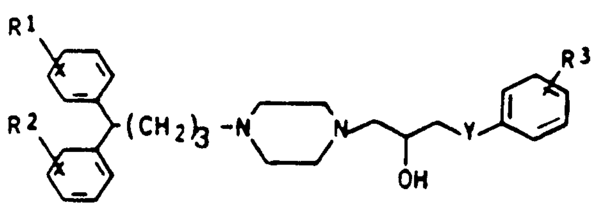

I

in which
$R^1$ and $R^2$ denote hydrogen, fluorine or chlorine atoms,
$R^3$ denotes a fluorine or chlorine atom, and
Y denotes an oxygen or sulfur atom,
and the salts thereof with physiologically tolerated acids.

2. 1-[4,4-bis(4-Fluorophenyl)butyl]-4-[2-hydroxy-3-(4-fluorophenoxy)propyl]piperazine.

3. (-)-1-[4,4-bis(4-Fluorophenyl)butyl]-4-[2-hydroxy-3-[4-fluorophenoxy)propyl]piperazine.

4. (+)-1-[4,4-bis(4-Fluorophenyl)butyl]-4-[2-hydroxy-3-(4-fluorophenoxy)propyl]piperazine.

5. A process for preparing the compounds of the formula I as claimed in claim 1, which comprises reacting

a) a compound of the formula II

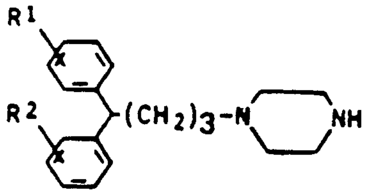

II

in which $R^1$ and $R^2$ have the stated meaning, with an epoxide of the formula III

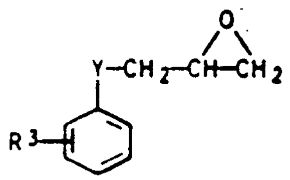

III

in which Y and $R^3$ have the stated meaning, or
b) a piperazine of the formula IV

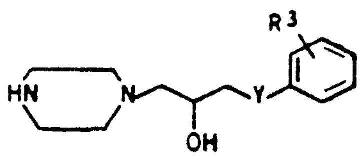

IV

in which Y and $R^3$ and n have the stated meaning, with a compound of the formula V

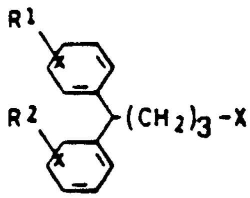

V

where $R^1$ and $R^2$ have the stated meaning, and X denotes a reactive acid residue, and converting the compounds obtained in this way where appropriate into their salts with physiologically tolerated acids.

**6.** A drug containing a compound of the formula I.

**7.** The use of a compound of the formula I for preparing a drug for the treatment of cardiovascular disorders and oxygen deficiency states of vital organs.

## Revendications

**1.** Pipérazines à substituant 2-hydroxy-3-phénoxypropyle, répondant à la formule I

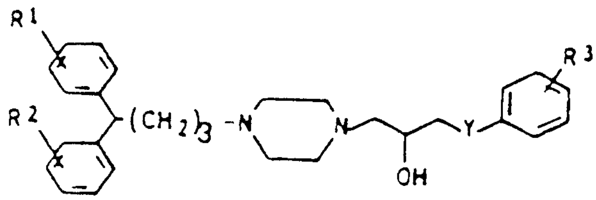

I

dans laquelle

| | |
|---|---|
| $R^1$ et $R^2$ | représentent des atomes d'hydrogène, de fluor ou de chlore, |
| $R^3$ | représente un atome de fluor ou de chlore et |
| Y | représente un atome d'oxygène ou de soufre, |

et leurs sels d'acides acceptables pour l'usage pharmaceutique.

**2.** 1-[4,4-bis(4-fluorophényl)butyl]-4-[2-hydroxy-3-(4-fluorophénoxy)propyl]pipérazine.

**3.** (-)-1-[4,4-bis(4-fluorophényl)butyl]-4-[2-hydroxy-3-[4-fluorophénoxy)propyl]pipérazine.

**4.** ( + )-1-[4,4-bis(4-fluorophényl)butyle]-4-[2-hydroxy-3-(4-fluorophénoxy)propyl]pipérazine.

**5.** Procédé de préparation des composés de formule I de la revendication 1, caractérisé en ce que

a) on fait réagir un composé de formule II

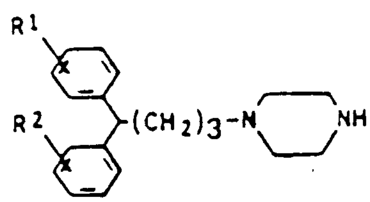

II

dans laquelle $R^1$ et $R^2$ ont les significations indiquées ci-dessus avec un époxyde de formule III

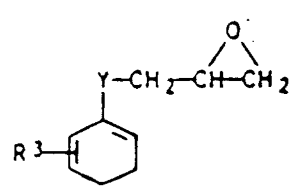

III

dans laquelle Y et $R^3$ ont les significations indiquées ci-dessus, ou bien

b) on fait réagir un pipérazine de formule IV

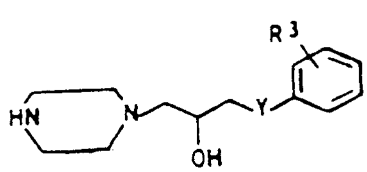

IV

dans laquelle Y et $R^3$ ont les significations indiquées ci-dessus, avec un composé de formule V

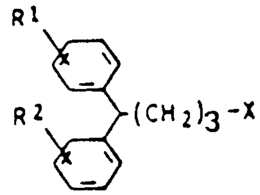

V

dans laquelle $R^1$ et $R^2$ ont les significations indiquées ci-dessus et Y représente un radical acide réactif,

et le cas échéant on convertit les composés ainsi obtenus en leurs sels d'acides acceptables pour l'usage pharmaceutique.

**6.** Médicament contenant un composé de formule I.

**7.** Utilisation d'un composé de formule I pour la préparation d'un médicament prévu pour le traitement des maladies cardiocirculatoires et des états de manque d'oxygène des organes vitaux.